# EUROPÄISCHE PATENTSCHRIFT

(11) **EP 2 705 075 B1**
(45) Veröffentlichungstag und Bekanntmachung des Hinweises auf die Patenterteilung: **21.12.2016**
(21) Anmeldenummer: 12718660.9
(22) Anmeldetag: 03.05.2012
(51) Int. Cl.: C08J 3/12, C08F 220/06, A61L 15/24, A61L 15/60, C08F 6/28

(54) **VERFAHREN ZUR HERSTELLUNG WASSERABSORBIERENDER POLYMERPARTIKEL**
METHOD FOR THE PRODUCTION OF WATER-ABSORBING POLYMER PARTICLES
PROCÉDÉ DE PRODUCTION DE PARTICULES POLYMÈRES ABSORBANT L'EAU

(30) Priorität: 06.05.2011 EP 11165153
(43) Veröffentlichungstag der Anmeldung: 12.03.2014
(73) Patentinhaber: BASF SE, 67056 Ludwigshafen am Rhein (DE)
(72) Erfinder: FUNK, Rüdiger, 65527 Niedernhausen (DE); PFEIFFER, Thomas, 67459 Böhl-Iggelheim (DE)
(74) Vertreter: BASF IP Association
(86) Internationale Anmeldenummer: PCT/EP2012/058112
(87) Internationale Veröffentlichungsnummer: WO 2012/152647

(56) Entgegenhaltungen:
- WO-A1-2007/057350
- WO-A1-2010/124954
- WO-A2-2010/066680

## Beschreibung

Die vorliegende Erfindung betrifft ein Verfahren zur Herstellung wasserabsorbierender Polymerpartikel, umfassend Polymerisation einer Monomerlösung oder -suspension, Trocknung des erhaltenen Polymergels, sowie Mahlung und Klassierung des getrockneten Polymergels, wobei die die Mahlung mittels eines mehrstufigen Walzenstuhls durchgeführt wird, der erste Mahlspalt eine Breite von 500 bis 5.000 µm aufweist, der den ersten Mahlspalt passierende Produktmassenstrom über einen Magnetabscheiders zum zweiten Mahlspalt geführt wird und der zweite Mahlspalt eine geringere Spaltbreite als der erste Mahlspalt aufweist.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm² (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die Abtrennung metallischer Verunreinigungen wird in WO 03/004450 A1, WO 2009/077376 A1, WO 2010/066680 A2 und WO 2010/124954 A1 beschrieben.

Aufgabe der vorliegenden Erfindung war die Bereitstellung eines verbesserten Verfahrens zur Herstellung wasserabsorbierender Polymerpartikel, insbesondere einer verbesserten Abtrennung metallischer Verunreinigungen vor der Mahlung.

Gelöst wurde die Aufgabe durch ein Verfahren zur Herstellung wasserabsorbierender Polymerpartikel durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

Trocknung des erhaltenen Polymergels, sowie Mahlung und Klassierung des getrockneten Polymergels, dadurch gekennzeichnet, dass die Mahlung mittels eines mehrstufigen Walzenstuhls durchgeführt wird, der erste Mahlspalt (Grobwalzenstuhl) eine Breite von 500 bis 5.000 µm aufweist, der den ersten Mahlspalt passierende Produktmassenstrom über einen Magnetabscheiders zum zweiten Mahlspalt geführt wird und der zweite Mahlspalt eine geringere Spaltbreite als der erste Mahlspalt aufweist.

Der erste Mahlspalt weist vorzugsweise eine Spaltbreite von 700 bis 3.000 µm, besonders bevorzugt von 850 bis 2.500 µm, ganz besonders von 1.000 bis 2.000 µm, auf.

Die im erfindungsgemäßen Verfahren einsetzbaren Magnetabscheider unterliegen keiner Beschränkung.

Die verwendeten Magnete weisen üblicherweise eine magnetische Flussdichte von mindestens 0,6 T, besonders bevorzugt von mindestens 0,9 T, ganz besonders bevorzugt von mindestens 1,1 T, auf.

Der Magnetabscheider ist vorzugsweise eine Rinne in der Form einer mit Rändern versehenen geneigten ebenen Fläche mit der effektiven Breite der Walzen, an deren Unterseite Magnete angebracht sind. Die Rinne bewirkt zusätzlich eine gleichmäßige Verteilung des vom ersten Mahlspalt kommenden Produktmassenstromes und eine gleichmäßige Beladung des zweiten Mahlspalts.

In dem erfindungsgemäßen Verfahren werden mehrstufige Walzenstühle eingesetzt, wobei vorzugsweise die Spaltbreite in Produktstromrichtung schrittweise verkleinert wird. Besonders bevorzugt sind dreistufige Walzenstühle. Mit steigender Anzahl der Stufen wird die Partikelgrößenverteilung enger. Ein mehrstufiger Walzenstuhl im Sinne dieser Erfindung ist beispielsweise ein Walzenstuhl mit mehreren hintereinander geschalteten Walzenpaaren oder mehrere hintereinander geschaltete Walzenstühle mit je einem Walzenpaar.

Bei der Trocknung wird das Polymergel üblicherweise auf Temperaturen oberhalb der Glasübergangstemperatur erhitzt. Daher sollte das getrocknete Polymergel vor der Mahlung unter die Glasübergangstemperatur abgekühlt und vorzerkleinert werden. Bei Verwendung von Umluftbandtrocknern geschieht dies üblicherweise im Anschluss an eine Kühlzone mittels einer integrierten Stachelwalze und einem Walzenbrecher.

Oft enthält der Produktmassenstrom noch unvollständig getrocknetes Polymergel. Dieses wird vorzugsweise mittels geeigneter Siebe, beispielsweise mit einer Maschenweite von 10 mm, vor der Mahlung abgetrennt, zerkleinert und nachgetrocknet. Vorteilhaft wird unvollständig getrocknetes Polymergel mittels zweier Siebe unterschiedlicher Maschenweite, beispielsweise 3 mm und 10 mm, abgetrennt, wobei nur die gröbere Partikelfraktion vor der Nachtrocknung zerkleinert wird.

Die Temperatur des der Mahlung zugeführten getrockneten Polymergels beträgt vorzugsweise von 30 bis 100°C, besonders bevorzugt von 35 bis 90°C, ganz besonders bevorzugt von 40 bis 80°C. Das getrocknete Polymergel zu weit abzukühlen ist unwirtschaftlich. Außerdem wird das Polymergel zu spröde, so dass der Anteil unerwünscht kleiner Polymerpartikel bei der Mahlung ansteigt. Ist das getrocknete Polymergel dagegen zu warm, so ist es wegen des geringen Abstandes zur Glasübergangstemperatur noch zu weich.

Die zur Mahlung üblicherweise eingesetzten Walzenstühle müssen vor dem Eintrag metallischer Verunreinigungen wie Schrauben oder Eisenspäne geschützt werden. Dies geschieht üblicherweise mittels eines Magnetabscheiders. Der vorliegenden Erfindung liegt nun die Erkenntnis zugrunde, dass sich derartige metallische Verunreinigungen besser nach einer Vorzerkleinerung in einem Grobwalzenstuhl entfernen lassen. Der Grobwalzenstuhl ist aufgrund des breiteren Mahlspaltes weniger empfindlich gegenüber diesen metallischen Verunreinigungen.

Im Folgenden wird die Herstellung der wasserabsorbierenden Polymerpartikel näher erläutert:
Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure,
0,0332 Gew.-% Wasser, 0,0203 Gew.-% Propionsäure, 0,0001 Gew.-% Furfurale,
0,0001 Gew.-% Maleinsäureanhydrid, 0,0003 Gew.-% Diacrylsäure und 0,0050 Gew.-% Hydro-chinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und
EP 0 937 736 A2 beschrieben, di- oder polyfunktionelle Alkohole, wie in DE 33 14 019 A1,
DE 35 23 617 A1 und EP 0 450 922 A2 beschrieben, oder ß-Hydroxyalkylamide, wie in
DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA empfohlenen Testmethode Nr. WSP 230.2-05 "Mass Loss Upon Heating" bestimmt wird.

Die gemäß dem erfindungsgemäßen Verfahren hergestellten wasserabsorbierenden Polymerpartikel haben einen Anteil an Partikeln mit einer Partikelgröße von 300 bis 600 µm von vorzugsweise mindestens 30 Gew.-%, besonders bevorzugt mindesten 50 Gew.-%, ganz besonders bevorzugt mindestens 70 Gew.-%.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA empfohlenen Testmethode Nr. WSP 242.2-05 "Absorption Under Pressure, Gravimetric Determination" bestimmt, wobei statt eines Drucks von 21,0 g/cm² ein Druck von 49,2 g/cm² eingestellt wird.

Die EDANA-Testmethoden sind beispielsweise erhältlich bei der EDANA, Avenue Eugene Plasky 157, B-1030 Brüssel, Belgien.

### Beispiele

89,5 g Acrylsäure, 805,6 g einer 37,3gew.-%igen wässrigen Natriumacrylatlösung, 97,3 g deionisiertes Wasser und 1,60 g 3-fach ethoxyliertes Glycerintriacrylat (ca. 85gew.-%ig) wurde für 30 Minuten durch Einleiten von Stickstoff von gelöstem Luftsauerstoff befreit. Die Polymerisation wurde in einem 2 Liter Kunststoffgefäß durch Zugabe von 1,5 g Natriumperoxodisulfat (30,0gew.-%ige wässrige Lösung), 2,0 g Ascorbinsäure (0,48gew.-%ige wässrige Lösung) und 2,5 g Wasserstoffperoxid (0,11gew.-%ige wässrige Lösung) gestartet.

800 g des Polymergels wurden mittels eines Extruders mit einer 6 mm Lochscheibe zerkleinert. 2,00 g Eisenspäne, die in ihrer Längsausrichtung unregelmäßig aber größtenteils spiralförmig gedreht waren, eine mittlere Länge von etwa 2 bis 5 mm (gestreckter Eisenspan) und eine mittlere Dicke von etwa 0,1 mm aufwiesen, wurden auf der flach ausgebreiteten Oberfläche von etwa einem Drittel des Polymergels (Schichtdicke des zerkleinerten Polymergels ca. 1 bis 2 cm) gleichmäßig verteilt. Darauf wurde zunächst ein zweites Drittel Polymergel mit vergleichbarer Schichtdicke geschichtet, erneut darauf 2,00 g Eisenspäne gleichmäßig verteilt und zuletzt mit dem dritten Drittel Polymergel wie zuvor beschichtet. Anschließend wurde das gesamte Polymergel vorsichtig manuell gemischt, so dass in keinem Bereich eine überdurchschnittliche Eisenspanansammlung zu beobachten war und dann erneut extrudiert.

Das mit Eisenspänen beladene Polymergel wurde 60 Minuten bei 150°C getrocknet und an einem Walzenstuhl mit einer eingestellten Spaltbreite von 2000 µm gemahlen. Erhalten wurden Polymerpartikel (Typ A), von denen ein Teil erneut am Walzenstuhl, diesmal jedoch mit einer eingestellten Spaltbreite von 1000 µm, zu Polymerpartikeln (Typ B) gemahlen wurden.

Die Polymerpartikel (Typ A) wurden über ein 1700 µm Sieb klassiert und die auf dem Sieb verbliebenen Partikel mit einem Magneten von den Partikeln ohne Eisenspan getrennt und als Partikel (Typ A1) für die nachfolgenden Versuche genutzt.

Die Polymerpartikel (Typ B) wurden über ein 1700 µm und ein 1000 µm Sieb klassiert und die auf dem 1000 µm Sieb verbliebenen Partikel mit einem Magneten von den Partikeln ohne Eisenspan getrennt und als Partikel (Typ B1) für die nachfolgenden Versuche genutzt.

Auf einer mit einem Steigungswinkel von 31° angeordneten, glatten Kunststoffplatte, wurden am oberen Ende der Platte über einen spaltförmigen Schacht auf einer Breite von 8 cm in einem Schwung Polymerpartikel (Typ A1 und B1) aus einer Höhe von 16 cm aufgetragen. In Abwärtsrichtung der Platte, beginnend ab 5,5 cm von der Auftragungslinie entfernt, waren über die gesamte Breite, die durch Seitenwände auf 11 cm begrenzt war, runde Magnete mit einem Durchmesser von 3 cm gestaffelt in drei dicht aufeinanderfolgende Reihen unterhalb der Kunststoffplatte angeordnet.

Die Masse der mittels des Magneten abgetrennten als auch die Masse der nicht abgetrennten Polymerpartikel wurde gravimetrisch bestimmt.

**Tab. 1: Bilanzierung der Polymerpartikel vom Typ A1**

| Einwaage [g] | abgetrennte Partikel [g] | nicht abgetrennte Partikel [g] | abgetrennte Partikel [%] | nicht abgetrennte Partikel [%] |
|---|---|---|---|---|
| 1,0321 | 0,6380 | 0,3942 | 61,8 | 38,2 |
| 1,0575 | 0,6476 | 0,4099 | 61,2 | 38,8 |
| 1,0057 | 0,6007 | 0,4053 | 59,7 | 40,3 |
| 1,0407 | 0,7156 | 0,3253 | 68,8 | 31,3 |
| 1,0457 | 0,6122 | 0,4333 | 58,5 | 41,4 |
| 1,0586 | 0,6062 | 0,4521 | 57,3 | 42,7 |
| 1,0365 | 0,5784 | 0,4577 | 55,8 | 44,2 |
| 1,0323 | 0,7861 | 0,2461 | 76,2 | 23,8 |
| 1,0339 | 0,6181 | 0,4158 | 59,8 | 40,2 |
| 1,0058 | 0,6300 | 0,3767 | 62,6 | 37,5 |
| Mittelwerte: | | | 62,2 | 37,8 |

**Tab. 2: Bilanzierung der Polymerpartikel vom Typ B1**

| Einwaage [g] | abgetrennte Partikel [g] | nicht abgetrennte Partikel [g] | abgetrennte Partikel [%] | nicht abgetrennte Partikel [%] |
|---|---|---|---|---|
| 1,0192 | 0,9296 | 0,0896 | 91,2 | 8,8 |
| 0,9927 | 0,8872 | 0,1056 | 89,4 | 10,6 |
| 1,0386 | 0,8760 | 0,1621 | 84,3 | 15,6 |
| 1,0461 | 0,8638 | 0,1825 | 82,6 | 17,4 |
| 1,0652 | 0,9423 | 0,1215 | 88,5 | 11,4 |
| 1,0441 | 0,8703 | 0,1708 | 83,4 | 16,4 |
| 1,0915 | 0,9656 | 0,1263 | 88,5 | 11,6 |
| 1,0589 | 0,9344 | 0,1247 | 88,2 | 11,8 |
| 1,0182 | 0,8975 | 0,1183 | 88,1 | 11,6 |
| 0,9988 | 0,8464 | 0,1525 | 84,7 | 15,3 |
| Mittelwerte: | | | 86,9 | 13,0 |

Am Ende der Versuchdurchführung wurden die Partikel zur Überprüfung ihrer Spanbeladung zerrieben. Es konnte bei > 95% der Partikel pro Partikel ein Eisenspan festgestellt werden.

Die Versuche belegen, dass die Abscheiderate steigt, wenn die Polymerpartikel mittels eines Grobwalzenstuhls vorzerkleinert werden.

## Patentansprüche

1. Verfahren zur Herstellung wasserabsorbierender Polymerpartikel durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
Trocknung des erhaltenen Polymergels, sowie Mahlung und Klassierung des getrockneten Polymergels, **dadurch gekennzeichnet, dass** die Mahlung mittels eines mehrstufigen Walzenstuhls durchgeführt wird, der erste Mahlspalt eine Spaltbreite von 500 bis 5.000 µm aufweist, der den ersten Mahlspalt passierende Produktmassenstrom über einen Magnetabscheiders zum zweiten Mahlspalt geführt wird und der zweite Mahlspalt eine geringere Spaltbreite als der erste Mahlspalt aufweist.

2. Verfahren gemäß Anspruch 1, **dadurch gekennzeichnet, dass** der mehrstufige Walzenstuhl mindestens drei unterschiedliche Mahlspalte aufweist.

3. Verfahren gemäß Anspruch 1 oder 2, **dadurch gekennzeichnet, dass** das Polymergel auf einem Umluftbandtrockner getrocknet wird.

4. Verfahren gemäß einem der Ansprüche 1 bis 3, **dadurch gekennzeichnet, dass** das getrocknete Polymergel vor der Mahlung vorzerkleinert wird.

5. Verfahren gemäß einem der Ansprüche 1 bis 4, **dadurch gekennzeichnet, dass** unvollständig getrocknetes Polymergel vor der Mahlung abgetrennt wird.

6. Verfahren gemäß einem der Ansprüche 1 bis 5, **dadurch gekennzeichnet, dass** das der Mahlung zugeführte getrocknete Polymergel eine Partikelgröße von höchstens 10 mm aufweist, wobei die Partikelgröße mit einem Sieb mit einer Maschenweite von 10 mm bestimmt wird.

7. Verfahren gemäß einem der Ansprüche 1 bis 6, **dadurch gekennzeichnet, dass** das der Mahlung zugeführte getrocknete Polymergel eine Temperatur von 40 bis 80°C aufweist.

8. Verfahren gemäß einem der Ansprüche 1 bis 7, **dadurch gekennzeichnet, dass** das Monomer a) zu mindestens 50 mol-% teilweise neutralisierte Acrylsäure ist.

9. Verfahren gemäß einem der Ansprüche 1 bis 8, **dadurch gekennzeichnet, dass** das Monomer a) zu 25 bis 85 mol-% neutralisiert ist.

10. Verfahren gemäß einem der Ansprüche 1 bis 9, **dadurch gekennzeichnet, dass** die wasserabsorbierenden Polymere eine Zentrifugenretentionskapazität von mindestens 15 g/g aufweisen, wobei die Zentrifugenretentionskapazität gemäß der von der EDANA empfohlenen Testmethode Nr. WSP 241.2-05 "Fluid Retention Capacity in Saline, After Centrifugaton" bestimmt wird.

## Claims

1. A process for producing water-absorbing polymer particles by polymerizing a monomer solution or suspension comprising
a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,
drying the resulting polymer gel, and grinding and classifying the dried polymer gel, wherein the grinding is performed by means of a multistage roll mill, the first milling gap has a gap width of 500 to 5000 µm, the product mass flow passing through the first milling gap is conducted through a magnetic separator to the second milling gap, and the second milling gap has a smaller gap width than the first milling gap.

2. The process according to claim 1, wherein the multistage roll mill has at least three different milling gaps.

3. The process according to claim 1 or 2, wherein the polymer gel is dried on a forced air belt drier.

4. The process according to any of claims 1 to 3, wherein the dried polymer gel is precomminuted before the grinding.

5. The process according to any of claims 1 to 4, wherein incompletely dried polymer gel is removed before the grinding.

6. The process according to any of claims 1 to 5, wherein the dried polymer gel supplied to the grinding has a particle size of at most 10 mm, the particle size being determined with a sieve having a mesh size of 10 mm.

7. The process according to any of claims 1 to 6, wherein the dried polymer gel supplied to the grinding has a temperature of 40 to 80°C.

8. The process according to any of claims 1 to 7, wherein monomer a) is acrylic acid partly neutralized to an extent of at least 50 mol%.

9. The process according to any of claims 1 to 8, wherein monomer a) has been neutralized to an extent of 25 to 85 mol%.

10. The process according to any of claims 1 to 9, wherein the water-absorbing polymers have a centrifuge retention capacity of at least 15 g/g, the centrifuge retention capacity being determined by EDANA recommended test method No. WSP 241.2-05, "Fluid Retention Capacity in Saline, After Centrifugation".

## Revendications

1. Procédé pour la préparation de particules polymères absorbant l'eau par polymérisation d'une solution ou d'une suspension de monomères, contenant
a) au moins un monomère éthyléniquement insaturé, portant des groupes acides, qui peut être au moins partiellement neutralisé,
b) au moins un réticulant,
c) au moins un initiateur,
d) éventuellement un ou plusieurs monomères éthyléniquement insaturés copolymérisables avec les monomères cités en a) et
e) éventuellement un ou plusieurs polymères solubles dans l'eau,
séchage du gel polymère obtenu, ainsi que broyage et classification du gel polymère séché, **caractérisé en ce que** le broyage est réalisé au moyen d'un broyeur à cylindres à plusieurs étages, dont la première fente de broyage présente une largeur de fente de 500 à 5000 µm, le flux massique de produit passant par la première fente de broyage est guidé via un séparateur magnétique vers une deuxième fente de broyage et la deuxième fente de broyage présente une largeur de fente plus petite que la première fente de broyage.

2. Procédé selon la revendication 1, **caractérisé en ce que** le broyeur à cylindres à plusieurs étages présente au moins trois fentes de broyage différentes.

3. Procédé selon la revendication 1 ou 2, **caractérisé en ce que** le gel polymère est séché sur un séchoir à bandes à circulation d'air.

4. Procédé selon l'une quelconque des revendications 1 à 3, **caractérisé en ce que** le gel polymère séché est prébroyé avant le broyage.

5. Procédé selon l'une quelconque des revendications 1 à 4, **caractérisé en ce que** le gel polymère incomplètement séché est séparé avant le broyage.

6. Procédé selon l'une quelconque des revendications 1 à 5, **caractérisé en ce que** le gel polymère séché introduit dans le broyage présente une grosseur de particule d'au plus 10 mm, la grosseur de particule étant déterminée à l'aide d'un tamis présentant une largeur de maille de 10 mm.

7. Procédé selon l'une quelconque des revendications 1 à 6, **caractérisé en ce que** le gel polymère séché introduit dans le broyage présente une température de 40 à 80°C.

8. Procédé selon l'une quelconque des revendications 1 à 7, **caractérisé en ce que** le monomère a) est, à raison d'au moins 50% en mole, de l'acide acrylique partiellement neutralisé.

9. Procédé selon l'une quelconque des revendications 1 à 8, **caractérisé en ce que** le monomère a) est neutralisé à raison de 25 à 85% en mole.

10. Procédé selon l'une quelconque des revendications 1 à 9, **caractérisé en ce que** les polymères absorbant l'eau présentent une capacité de rétention à la centrifugeuse d'au moins 15 g/g, la capacité de rétention à la centrifugeuse étant déterminée selon le procédé de test recommandé par l'EDENA n° WSP 241.2-50 "Fluid Retention Capacity in Saline, After Centrifugation".
